# EUROPEAN PATENT APPLICATION

(11) **EP 4 471 125 A1**
(43) Date of publication of application: **04.12.2024**
(21) Application number: 23743340.4
(22) Date of filing: 20.01.2023
(51) Int. Cl.: C12N 5/076, C12N 5/0775, C12N 1/00

(54) **COMPOSITION FOR SPERMATOZOA TREATMENT, SPERMATOZOA MOTILITY ENHANCEMENT AGENT, SPERMATOZOA FERTILITY RETENTION AGENT, SPERMATOZOA MOTILITY ENHANCEMENT METHOD, AND SPERMATOZOA FERTILITY RETENTION METHOD**

(30) Priority: 24.01.2022 JP 2022008746; 28.10.2022 JP 2022173215
(71) Applicant: Hiroshima University, Higashihiroshima-shi Hiroshima 739-8511 (JP); Rohto Pharmaceutical Co., Ltd., Osaka-shi, Osaka 544-8666 (JP)
(72) Inventor: SHIMADA Masayuki, Higashi-Hiroshima City, Hiroshima 739-8511 (JP); UMEHARA Takashi, Higashi-Hiroshima City, Hiroshima 739-8511 (JP); ISHIKAWA Noriyasu, Soraku-gun, Kyoto 619-0237 (JP); TAKIJIRI Takashi, Osaka-shi, Osaka 544-8666 (JP)
(74) Representative: Mewburn Ellis LLP
(86) International application number: PCT/JP2023/001663
(87) International publication number: WO 2023/140348

(57) **Abstract**

The purpose of the present invention is to provide spermatozoa having high motility. The present invention is a composition for treating sperm that comprises a mesenchymal stem cell culture supernatant. The composition for spermatozoa treatment of the present invention is used as a sperm preparing solution, a sperm diluting solution, a sperm storage solution, an artificial insemination solution, an in vitro fertilization solution, a solution for improving sperm motility, or a solution for retaining sperm fertilizing capabilities. Furthermore, mesenchymal stem cells in the mesenchymal stem cell culture supernatant comprised in the composition for treating sperm are preferably derived from adipose tissue, derived from umbilical cord tissue, or derived from bone marrow tissue.

## Description

### TECHNICAL FIELD

The present invention relates to a composition for treating sperm, an agent for improving sperm motility, an agent for retaining sperm fertility, a method for improving sperm motility, and a method for retaining sperm fertility.

### BACKGROUND ART

"Infertility" refers to the inability to conceive for a certain period of time despite sexual intercourse without contraception between a healthy man and woman who wish to conceive, and this "certain period of time" is defined as "generally one year" by the Japan Society of Obstetrics and Gynecology. It is estimated that about one in ten couples are infertile, but in recent years, the age at which people think about conception has been rising, and it is known that both men and women become less likely to become pregnant as they age, and thus it is said that infertility rate is even higher.

Infertility may be caused by the male, by the female, or both, or by unknown cause, but it is said that about half of all cases the cause is also on the male side. Examples of a known cause of such male infertility include a sexual dysfunction such as spermatogenesis dysfunction, obstruction of sperm transport, erectile dysfunction (ED), or intravaginal ejaculation dysfunction, and aging. In men, it is said that the quality of sperm gradually deteriorates from around the age of 35. As people have been getting married later in life in recent years, it is said that about 10% of men have a problem with their semen findings even before marriage.

Infertility treatment is carried out by selecting the optimal treatment method according to the cause. Major treatment methods include a timing method, an ovulation induction method, artificial insemination, and even an assisted reproductive technique such as in vitro fertilization. Artificial insemination (AIH) is a treatment method involving injecting sperm into the uterine cavity in order to deliver sufficient sperm necessary for fertilization to the ampulla of the fallopian tube, which is the site of fertilization. AIH is indicated for oligozoospermia (sperm concentration of 15 million/ml or less), asthenospermia (motility rate of 40% or less), sexual intercourse dysfunction, sperm cervical mucus incompatibility (failing the Huhner test), a case of anti-sperm antibody retention, and a case of unexplained infertility. However, it is said that the limit of AIH is a total motile sperm count after treating of 1 million to 5 million, and there are also many cases in which it is difficult to satisfy this limit. In addition, when frozen semen is used for artificial insemination, the motility of the sperm decreases because of freezing, and the conception rate cannot currently be said to be high. Therefore, development of a method for enhancing the motility of sperm is desired.

Mesenchymal stem cells are multipotent progenitor cells that were first isolated from bone marrow by Friedenstein (1982) (see Non Patent Document 1). It has been revealed that mesenchymal stem cells are present in various tissues such as bone marrow, umbilical cord, and fat, and mesenchymal stem cell transplantation is expected as a new therapeutic method for various intractable diseases (see Patent Documents 1 and 2). Recently, it has been known that cells having comparable functions are present in stromal cells in adipose tissue, placenta, umbilical cord, egg membrane, and the like. Therefore, mesenchymal stem cells are sometimes called mesenchymal stromal cells.

### PRIOR ART DOCUMENTS

### Patent Document

Patent Document 1: JP 2012-157263 A
Patent Document 2: JP 2012-508733 A

### Non Patent Document

Non Patent Document 1: Pittenger F. M. et al., Science 284, pp.143-147, 1999

### SUMMARY OF INVENTION

### Technical Problem

Under the circumstances described above, an object of the present invention is to provide sperm having excellent motility.

### Solution to Problem

The present inventors have carried out intensive research in order to solve the above problem and as a result, found that a culture supernatant of mesenchymal stem (stromal) cells (MSCs) increases the motion performance of sperm, and completed the present invention. According to the present invention, sperm having high motility and suppressed sperm acrosome reaction can be provided. That is, the gist of the present invention is as follows.
[1] A composition for treating sperm, comprising a mesenchymal stem cell culture supernatant.
[2] The composition for treating sperm according to [1], wherein the composition is a sperm conditioning solution, a sperm dilution solution, a sperm preservation solution, an artificial insemination solution, an in vitro fertilization solution, a solution for improving sperm motility, or a solution for retaining sperm fertility.
[3] The composition for treating sperm according to [1] or [2], wherein the mesenchymal stem cells are derived from adipose tissue, umbilical cord tissue, or bone marrow tissue.
[4] The composition for treating sperm according to any one of [1] to [3], wherein the composition is a mesenchymal stem cell culture supernatant obtained by culturing the mesenchymal stem cells in a serum-free medium.
[5] An agent for improving sperm motility, comprising a mesenchymal stem cell culture supernatant.
[6] An agent for retaining sperm fertility, comprising a mesenchymal stem cell culture supernatant.
[7] A method for improving sperm motility, comprising culturing sperm in a solution comprising a mesenchymal stem cell culture supernatant.
[8] A method for retaining sperm fertility, comprising culturing sperm in a solution comprising a mesenchymal stem cell culture supernatant.

### Advantageous Effects of Invention

According to the present invention, sperm having high motility and suppressed sperm acrosome reaction can be provided. The sperm of the present invention have higher motility than and suppressed sperm acrosome reaction as compared with sperm treated by a conventional method, and thus have an elevated fertilization success rate.

### BRIEF DESCRIPTION OF DRAWINGS

[Fig. 1] Fig. 1 is a diagram showing the motility of mouse sperm under each condition.
[Fig. 2] Fig. 2 is a diagram showing results of counting sperm acrosome marker positive cells under each condition by flow cytometry.
[Fig. 3] Fig. 3 is a diagram showing the proportion of acrosome marker positive sperm under each condition.
[Fig. 4] Fig. 4 is a diagram showing the extent of increase in the motility of mouse sperm under each condition.
[Fig. 5] Fig. 5 is a diagram showing the motility of mouse sperm under each condition.
[Fig. 6] Fig. 6 is a diagram showing the proportion of acrosome marker positive sperm under each condition.
[Fig. 7] Fig. 7 is a diagram showing the cleavage rate under each condition.
[Fig. 8] Fig. 8 is a diagram showing the proportion of motile sperm in cattle sperm under each condition.
[Fig. 9] Fig. 9 is a diagram showing the proportion of motile sperm in cattle sperm under each condition.
[Fig. 10] Fig. 10 is a diagram showing the motility of cattle sperm under each condition.
[Fig. 11] Fig. 11 is a diagram showing the extent of increase in the motility of cattle sperm under each condition.
[Fig. 12] Fig. 12 is a diagram showing the proportion of motile sperm in all sperm observed for pig sperm under each condition.
[Fig. 13] Fig. 13 is a diagram showing the motility of pig sperm under each condition.

### DESCRIPTION OF EMBODIMENTS

Hereinafter, the composition for treating sperm, the agent for improving sperm motility, the agent for retaining sperm fertility, the method for improving sperm motility, and the method for retaining sperm fertility according to the present invention will be described in detail.

### [Composition for treating sperm]

The composition for treating sperm according to the present invention is characterized by including a mesenchymal stem cell culture supernatant. The composition for treating sperm according to the present invention can provide sperm having high motility and suppressed sperm acrosome reaction by including a mesenchymal stem cell culture supernatant. Sperm treated with the composition for treating sperm according to the present invention and having improved or increased motility and suppressed acrosome reaction can be expected to, for example, increase fertilization efficiency or conception rate in artificial insemination or in vitro fertilization, or promote early development. The composition for treating sperm according to the present invention may include a further component as long as the further component does not impair the effect of the present invention, in addition to the mesenchymal stem cell culture supernatant, which is an essential component.

The motility of sperm can be expressed by a sperm motion parameter. Examples of such a motion parameter include the amplitude of the lateral oscillatory movement of the sperm head (amplitude of lateral head displacement; ALH), the number of lateral oscillatory movements of the sperm head (beat cross frequency; BCF), the velocity based on the total distance traveled by sperm (curvilinear velocity; VCL), and the velocity based on the straight-line distance traveled by sperm (straight-line velocity; VSL). ALH/VCL (value obtained by dividing ALH by VCL) indicates the degree of motility in zigzag movement characteristic of sperm. In addition, BCF/VSL (value obtained by dividing BCF by VSL) indicates the degree of motility in straight-line movement of sperm.

### (Mesenchymal stem cell culture supernatant)

In the present invention, the mesenchymal stem cell culture supernatant is a culture supernatant obtained when mesenchymal stem cells are cultured.

In the present invention, mesenchymal stem cells mean cells that have the ability to differentiate into one or more types of cells belonging to the mesenchymal system (osteocytes, cardiomyocytes, chondrocytes, tendon cells, adipocytes, and the like) and that can proliferate while maintaining this ability. The term mesenchymal stem cells used in the present invention means the same cells as stromal cells, and there is no particular distinction between the two. In addition, the mesenchymal stem cells are also sometimes simply referred to as mesenchymal cells. Examples of a tissue including mesenchymal stem cells include adipose tissue, umbilical cord, bone marrow, umbilical cord blood, endometrium, placenta, amnion, chorion, decidua, dermis, skeletal muscle, periosteum, dental follicle, periodontal ligament, dental pulp, and tooth germ. Examples of the mesenchymal stem cells in the present invention include those derived from adipose tissue, umbilical cord, bone marrow, umbilical cord blood, endometrium, placenta, amnion, chorion, decidua, dermis, skeletal muscle, periosteum, dental follicle, periodontal ligament, dental pulp, tooth germ, and the like, and among these, adipose tissue-derived mesenchymal stem cells, umbilical cord-derived mesenchymal stem cells, and bone marrow-derived mesenchymal stem cells are preferable, and adipose tissue-derived mesenchymal stem cells and umbilical cord-derived mesenchymal stem cells are more preferable.

Examples of species of the mesenchymal stem cells in the present invention include a human, a horse, cattle, a sheep, a pig, a dog, a cat, a rabbit, a mouse, and a rat.

The mesenchymal stem cells in the present invention may be derived from the same species as the target to be treated (subject), or derived from a different species therefrom.

The mesenchymal stem cells may be cells provided by, for example, PromoCell, Lonza, Biological Industries, Veritas, R&D Systems, or Corning, or cells prepared by a method well known to those skilled in the art. In addition, the mesenchymal stem cells may be primary cells separated from a donor tissue, or cells established as a cell line.

The medium used to culture mesenchymal stem cells in the present invention is not particularly limited as long as the medium is a medium that allows mesenchymal stem cells to be cultured while keeping the mesenchymal stem cells in good condition, and is preferably, for example, a medium that allows human mesenchymal stem cells to proliferate while maintaining the ability thereof to differentiate into osteocytes, chondrocytes, and adipocytes.

The medium used in the present invention may be created by supplementing a basal medium with a serum and/or with one or more serum substitutes such as albumin, transferrin, a fatty acid, insulin, sodium selenite, cholesterol, a collagen precursor, a trace element, 2-mercaptoethanol, or 3'-thiolglycerol. In addition, if necessary, such a medium may be further supplemented with a substance such as an amino acid such as glutamine, a sugar such as glucose, a metal salt such as sodium chloride or magnesium sulfate, a trace metal such as selenium, a lipid such as cholesterol or an unsaturated fatty acid, a vitamin such as pantothenic acid, a protein such as albumin, insulin, transferrin, a growth factor, a proliferation factor, or a cytokine, a polysaccharide, a low-molecular-weight compound, an antibiotic, an antioxidant, a pyruvic acid, a buffer, or an inorganic salt.

Examples of the basal medium include IMDM medium, Medium 199 medium, Eagle's Minimum Essential Medium (EMEM) medium, αMEM medium, Dulbecco's modified Eagle's Medium (DMEM) medium, Ham's F12 medium, RPMI 1640 medium, Fischer 's medium, MCDB201 medium, and a mixed medium thereof.

From the viewpoint of use for treating sperm, the medium for mesenchymal stem cells is preferably a xeno-free medium, which is free of a xeno-derived component such as a serum. Examples of such a medium include a medium provided as a pre-prepared medium for mesenchymal stem cells (stromal cells) such as Mesenchymal Stem Cell Growth Medium 2 (Ready-to-use, manufactured by PromoCell), Mesenchymal Stem Cell Growth Medium XF (Ready-to-use, manufactured by PromoCell), MSCGM BulletKittm, MSCGMtm Mesenchymal Stem Cell Growth Medium BulletKittm (manufactured by Lonza), a xeno-free medium for human mesenchymal stem cells (MSC NutriStem (registered trademark) XF, manufactured by Biological Industries), MesenCult-ACF Plus (manufactured by Veritas), StemXVivotm Serum-Free Human MSC Expansion Media (manufactured by R&D Systems, Corning), a serum-free medium for adipose-derived stem cells (KBM ADSC-4, manufactured by Kohjin Bio), and a serum-free medium for mesenchymal stem cells (R: STEM Medium for hMSC High Growth, manufactured by Rohto).

Examples of the serum described above include, but are not limited to, human serum, fetal bovine serum (FBS), bovine serum, calf serum, goat serum, horse serum, pig serum, sheep serum, rabbit serum, and rat serum. When a serum is used, a basal medium may be supplemented with 5 v/v% to 15 v/v%, preferably 10 v/v%, thereof.

Examples of the fatty acid include, but are not limited to, linoleic acid, oleic acid, linolenic acid, arachidonic acid, myristic acid, palmitoyl acid, palmitic acid, and stearic acid. Examples of the lipid include, but are not limited to, phosphatidylserine, phosphatidylethanolamine, and phosphatidylcholine. Examples of the amino acid described above include, but are not limited to, L-alanine, L-arginine, L-aspartic acid, L-asparagine, L-cysteine, L-cystine, L-glutamic acid, L-glutamine, and L-glycine. Examples of the protein include, but are not limited to, ecotin, reduced glutathione, fibronectin, and B2-microglobulin. Examples of the polysaccharide include, but are not limited to, a glycosaminoglycan, and particular examples of the glycosaminoglycan include, but are not limited to, hyaluronic acid and heparan sulfate. Examples of the proliferation factor include, but are not limited to, platelet-derived growth factor (PDGF), basic fibroblast growth factor (bFGF), transforming growth factor beta (TGF-B), hepatocyte growth factor (HGF), epidermal growth factor (EGF), connective tissue growth factor (CTGF), and vascular endothelial growth factor (VEGF).

The medium used to obtain a culture supernatant of mesenchymal stem cells in the present invention is preferably a medium for mesenchymal stem cells supplemented with at least one selected from the group consisting of EGF, bFGF, albumin, transferrin, and insulin, more preferably a medium for mesenchymal stem cells including at least two selected therefrom, further preferably a medium for mesenchymal stem cells including at least three selected therefrom, and particularly preferably a medium for mesenchymal stem cells including at least four selected therefrom, in addition to a basal medium, and most preferably a medium for mesenchymal stem cells including EGF, bFGF, albumin, transferrin, and insulin in addition to a basal medium.

### (Preparation of mesenchymal stem cell culture supernatant)

The supernatant of mesenchymal stem cells obtained by the following method can be used as the mesenchymal stem cell culture supernatant in the present invention. In addition, the supernatant having an unnecessary component removed therefrom by means such as dialysis or ultrafiltration, a fraction obtained by fractionating the supernatant with a column or the like, a fraction selected by using an antibody against a specific molecule, a fraction obtained by a centrifugal operation, or the like may be used as the mesenchymal stem cell culture supernatant in the present invention.

As the medium used to obtain the culture supernatant, the same medium as the medium for culturing mesenchymal stem cells can be used. The method for obtaining the culture supernatant is not particularly limited as long as the method is a method suitable for culturing each mesenchymal stem cell, and is, for example, a method for culturing mesenchymal stem cells at a temperature of 20°C to 37°C, under an environment of 2% to 7% CO₂, and under an environment of 5% to 21% O₂, and preferably at room temperature to 37°C and under an environment of 5% CO₂ to obtain a culture supernatant thereof.

For the mesenchymal stem cell culture supernatant of the present invention, mesenchymal stem cells and a medium may be in contact with each other, and the washing solution obtained by washing mesenchymal stem cells with a medium can also be used as the culture supernatant of the present invention. The contact time between the mesenchymal stem cells and the medium is, for example, 14 days or less, preferably 10 days or less, further preferably 7 days or less, and more preferably 5 days or less. In addition, the preferred contact time between the mesenchymal stem cells and the medium is 10 seconds to 14 days, 30 seconds to 10 days, 1 minute to 7 days, 5 minutes to 7 days, 10 minutes to 7 days, 15 minutes to 7 days, 30 minutes to 7 days, 30 minutes to 6 days, or 30 minutes to 5 days. Culture for obtaining the culture supernatant may be a planar culture carried out by adhesion to a flask, or a floating/agitated culture involving adhesion to a microbead or the like.

The amount of the mesenchymal stem cell culture supernatant included in the composition for treating sperm according to the present invention is 0.1% by weight to 100% by weight, preferably 0.5% by weight to 95% by weight, more preferably 1% by weight to 90% by weight, further preferably 3% by weight to 80% by weight, and particularly preferably 5% by weight to 50% by weight, of the whole composition for treating sperm. By setting the amount of the mesenchymal stem cell culture supernatant included in the composition for treating sperm according to the present invention within the above numerical range, the composition for treating sperm according to the present invention is excellent in the effect of increasing the motility of sperm and suppressing the acrosome reaction of sperm.

The composition for treating sperm according to the present invention may contain a further component as long as the further component does not impair the effect of the present invention, in addition to the mesenchymal stem cell culture supernatant. Examples of the further component include a protective agent such as dimethylsulfoxide (DMSO) or serum albumin, an antibiotic, a vitamin, a carrier, an excipient, a disintegrant, a buffer, an emulsifier, a stabilizer, a preservative, an antiseptic, and physiological saline. In addition, a buffer solution that is commercially available as a buffer solution (washing solution) for an ovum or sperm or the like may be used. Examples of such a buffer solution include HTF Medium (manufactured by FUJIFILM Wako Pure Chemical Corporation) containing NaCl, KCl, KH₂PO₄, MgSO₄·7H₂O, CaC1₂·2H₂O, NaHCO₃, Glucose, Na-pyruvate, Na-lactate, Phenol Red, Hepes, Gentamicin Sulfate salt, and the like.

The species of sperm that are the target of the composition for treating sperm according to the present invention may be any mammalian animal, and examples thereof include a human, a horse, cattle, a sheep, a pig, a dog, a cat, a rabbit, a mouse, a rat, and a rare animal.

The composition for treating sperm according to the present invention is used as a sperm conditioning solution, a sperm dilution solution, a sperm preservation solution, an artificial insemination solution, an in vitro fertilization solution, a solution for improving sperm motility, a solution for retaining sperm fertility, or the like.

The sperm conditioning solution is a solution for washing sperm and even suspending sperm to recover sperm that show good motility, when providing sperm for the purpose of artificial insemination, in vitro fertilization, or the like. The sperm dilution solution is a solution used to dilute sperm to an appropriate concentration and inject the same into the uterus when carrying out artificial insemination. The sperm preservation solution is a solution that can be used for chilled preservation or frozen preservation of sperm. The artificial insemination solution is a solution used to increase the fertilization rate in artificial insemination. The in vitro fertilization solution is a solution used to increase the fertilization rate in in vitro fertilization. The solution for improving sperm motility is a solution used to improve the motility of sperm. The solution for retaining sperm fertility is a solution used to retain the fertility of sperm.

The composition for treating sperm according to the present invention can be prepared by a conventional method after mixing a further necessary component into the medium for mesenchymal stem cells described above.

### [Agent for improving sperm motility and agent for retaining sperm fertility]

The agent for improving sperm motility and the agent for retaining sperm fertility according to the present invention are characterized by including a mesenchymal stem cell culture supernatant. The agent for improving sperm motility and the agent for retaining sperm fertility according to the present invention can provide sperm having increased sperm motility, suppressed acrosome reaction, and retained fertility by including a mesenchymal stem cell culture supernatant. Sperm treated with the agent for improving sperm motility and the agent for retaining sperm fertility according to the present invention and thereby having increased motility and suppressed acrosome reaction and can be expected to, for example, increase fertilization efficiency or conception rate in artificial insemination or in vitro fertilization, or promote early development.

The agent for improving sperm motility and the agent for retaining sperm fertility according to the present invention are substantially the same as the composition for treating sperm described above, and thus for a specific description thereof, the description in the section Composition for treating sperm can be applied as it is by replacing the composition for treating sperm with the agent for improving sperm motility or the agent for retaining sperm fertility.

In a method for treating sperm with the agent for improving sperm motility and the agent for retaining sperm fertility according to the present invention, a state in which the agent for improving sperm motility and the agent for retaining sperm fertility are in contact with sperm is formed in vivo or in vitro. In vivo, it is considered to administer the agent for improving sperm motility or the agent for retaining sperm fertility to the seminal vesicle, which is an organ for storing sperm. As long as a state in which the agent for improving sperm motility or the agent for retaining sperm fertility according to the present invention is in contact with sperm is formed after administration, the administration site and the administration method are not particularly limited. On the other hand, in order to in vitro form a state in which the agent for improving sperm motility or the agent for retaining sperm fertility according to the present invention is in contact with sperm, for example, previously provided sperm and the agent for improving sperm motility or the agent for retaining sperm fertility according to the present invention are placed and mixed in an appropriate container in vitro and treated for a certain period of time. The treatment temperature is 4°C to 40°C, preferably 15°C to 40°C, more preferably 25°C to 39°C, and further preferably 36°C to 38°C, which is about the same as body temperature. The treatment time is 10 minutes to 48 hours, preferably 20 minutes to 24 hours, more preferably 30 minutes to 4 hours, and further preferably 1 hour to 2 hours.

Sperm treated with the agent for improving sperm motility or the agent for retaining sperm fertility according to the present invention can be used for artificial insemination or in vitro fertilization (increased success rate or efficiency), breeding of livestock (increased success rate or efficiency of artificial insemination, breeding or maintenance of species (for example, maintenance of endangered species, or maintenance or crossbreeding of a pet strain), treatment or improvement of various diseases associated with sperm disorder or caused mainly or secondarily by sperm disorder (for example, varicocele, male infertility, cryptorchism, X-ray irradiation, or sperm disorder after malignant tumor surgery or chemotherapy), preservation of sperm, improvement of the motility, retention of the fertility, or preservation of immature sperm, and the like.

When artificial insemination is carried out by using sperm having motility improved and fertility retained by the agent for improving sperm motility or the agent for retaining sperm fertility according to the present invention (also including artificial insemination for the purpose of breeding of livestock, or breeding or maintenance of species), a state in which sperm treated to have improved motility and/or retained fertility and an ovum (egg cell, egg) coexist is formed in vivo or in vitro. In order to form the coexistence state in vivo, sperm having improved motility and/or retained fertility are administered to (implanted into) a site in a living organism where an ovum is present. For example, in the case of a mammalian animal, sperm having improved motility and/or retained fertility are injected into the uterus. On the other hand, if the coexistence state is formed in vitro, an ovum provided, for example, by isolation from a living organism or distribution from a cell bank or the like and sperm having improved motility and/or retained fertility may be placed in the same container and incubated, or sperm having improved motility and/or retained fertility may be inserted into the ovum by using a micromanipulator or the like. A conventional method can be followed except for using the condition characteristic of the present invention, that is, using sperm having motility improved and/or fertility retained by the agent for improving sperm motility or the agent for retaining sperm fertility according to the present invention.

### [Method for improving sperm motility and method for retaining sperm fertility]

The present invention also includes a method for improving sperm motility and a method for retaining sperm fertility, characterized by culturing sperm in a solution including a mesenchymal stem cell culture supernatant. The method for improving sperm motility and the method for retaining sperm fertility according to the present invention can provide sperm having increased motility, improved motility, and/or retained fertility while maintaining the acrosome by culturing sperm in a solution including a mesenchymal stem cell culture supernatant. Sperm having motility increased and acrosome reaction suppressed by the method for improving sperm motility or the method for retaining sperm fertility according to the present invention can be expected to, for example, increase fertilization efficiency or conception rate in artificial insemination or in vitro fertilization, or promote early development.

The method for improving sperm motility and the method for retaining sperm fertility according to the present invention are methods for treating sperm with the agent for improving sperm motility or the agent for retaining sperm fertility according to the present invention described above, and thus for a specific description thereof, the section Agent for improving sperm motility and agent for retaining sperm fertility can be referred to.

### EXAMPLES

Hereinafter, the present invention will be described in detail with reference to Examples and Test Examples, but the present invention is not limited by these Examples and the like.

### [Example 1: Preparation of culture supernatant (1)]

Umbilical cord mesenchymal stem cells (C-12971 Human Mesenchymal Stem Cells from Umbilical Cord Matrix (hMSC-UC), manufactured by PromoCell) were cultured in each of RIM medium (serum-free medium for mesenchymal stem cells, containing EGF, bFGF, albumin, transferrin, and insulin, Rohto) and RS medium (serum-free medium for mesenchymal stem cells, containing EGF, bFGF, albumin, transferrin, and insulin, Rohto) for 3 days to obtain culture supernatants (hereinafter referred to as "RIM culture supernatant D3" and "RS culture supernatant D3," respectively). Various factors included in the culture supernatants were measured by ELISA, and it was confirmed that PGE2, IL-6, HGF, MCP1, MMP2, BDNF, and CD63+ exosomes were included.

### [Example 2: Motility of sperm (1)]

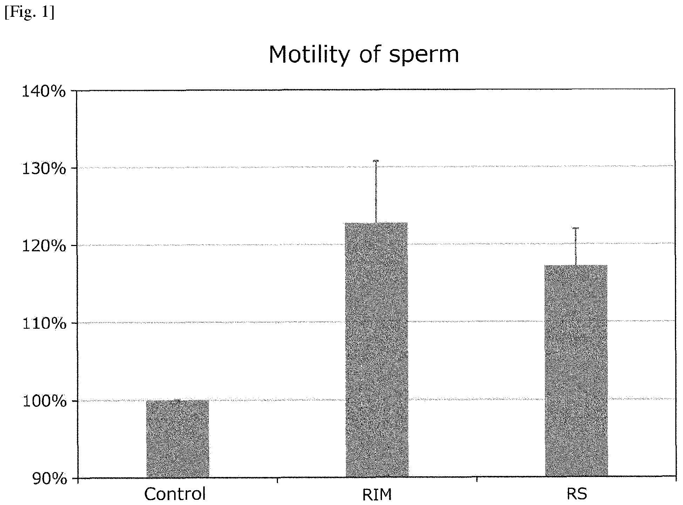

Mouse sperm (C57/BL6, 12 weeks old) were suspended in HTF Medium (manufactured by FUJIFILM Wako Pure Chemical Corporation) supplemented with RIM culture supernatant D3 or RS culture supernatant D3 in such a way as to have a final concentration of 10% (hereinafter also referred to as 10% RIM culture supernatant D3 or 10% RS culture supernatant D3, respectively), and HTF Medium supplemented with no culture supernatant as a negative control (control) in a water bath at 37°C, and these were each used as a sample. Subsequently, the sample was cultured for 1 hour at 37°C. Next, 3 µL of the sample was analyzed by using a sperm motility analyzer (Computer Assisted Sperm Analysis (CASA) system). The straight-line velocity (VSL) of all sperm observed was analyzed. With the median value for the control set to 100, the median values for 10% RIM culture supernatant D3 (RIM) and 10% RS culture supernatant D3 (RS) are shown in Fig. 1.

As shown in Fig. 1, it was confirmed that the supplementation with the cell culture supernatant (10% RIM culture supernatant D3, 10% RS culture supernatant D3) increased the median value and increased the motility of the sperm.

### [Example 3: Suppression of acrosome reaction of sperm (1)]

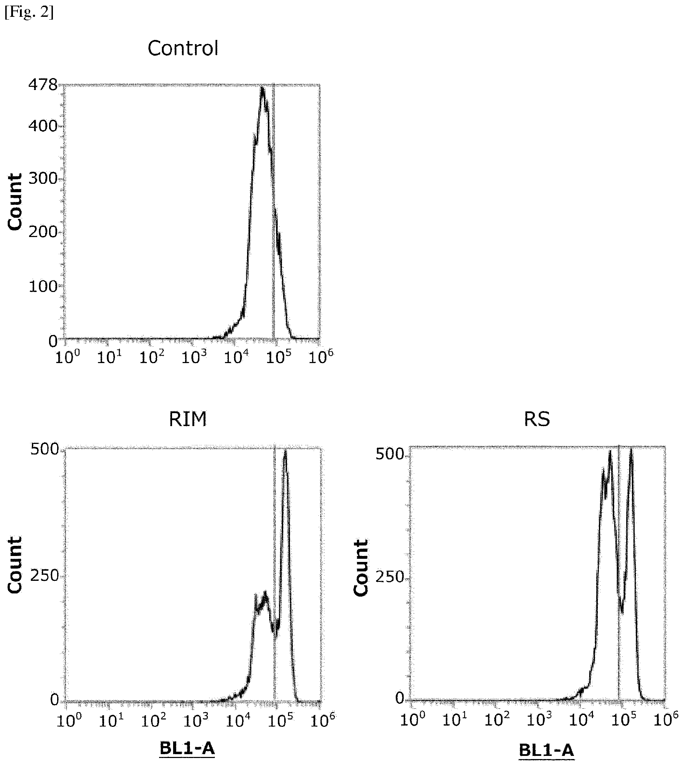

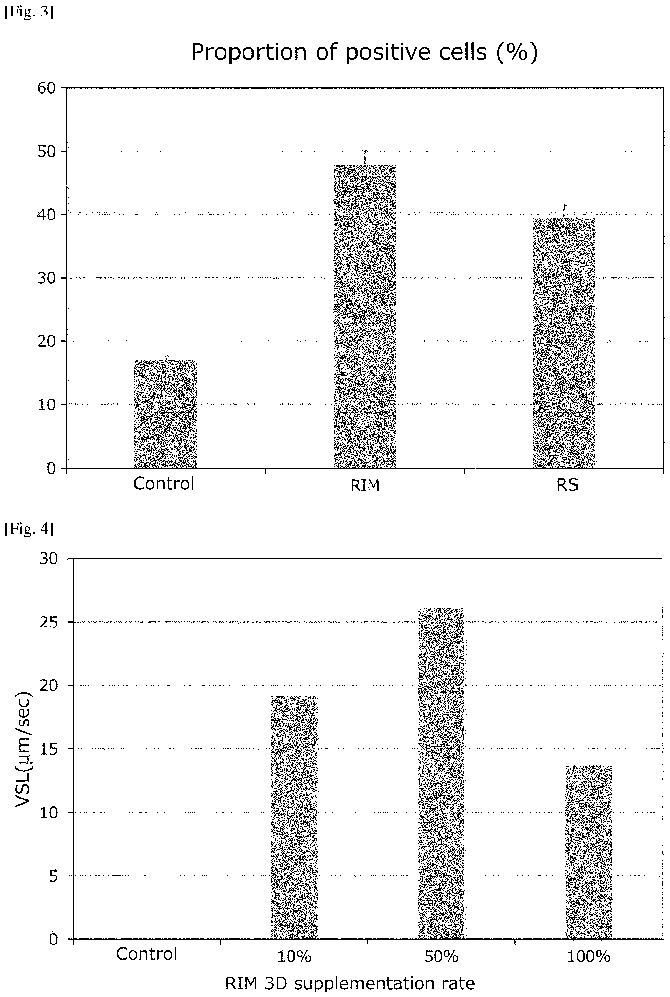

Sperm were collected from the epididymis of a male mouse (C57/BL6, 12 weeks old) and suspended in HTF Medium (manufactured by FUJIFILM Wako Pure Chemical Corporation) supplemented with RIM culture supernatant D3 or RS culture supernatant D3 in such a way as to have a final concentration of 10% and HTF Medium supplemented with no culture supernatant as a negative control (control), and after culture for 60 minutes, the proportion of sperm retaining an acrosome was analyzed by counting positive sperm by flow cytometry. Histograms of the number of positive sperm retaining an acrosome are shown in Fig. 2, and the proportions of the positive sperm are shown in Fig. 3.

As shown in Fig. 2 and Fig. 3, it was confirmed that the supplementation with the cell culture supernatant remarkably suppressed the acrosome reaction (RIM, RS). Therefore, it was revealed that the loss of fertility of sperm can be suppressed by supplementing a sperm medium such as HTF Medium with a cell culture supernatant such as RIM culture supernatant D3 or RS culture supernatant D3. When the culture supernatant obtained by culturing umbilical cord mesenchymal stem cells (C-12971 Human Mesenchymal Stem Cells from Umbilical Cord Matrix (hMSC-UC), manufactured by PromoCell) in RIM medium for 1 day or 4 days was used to examine the motility of sperm and suppression of the acrosome reaction of sperm, the effect of improving the motility of sperm and the effect of suppressing the loss of fertility of sperm were observed as in the above test.

### [Example 4: Motility of sperm (2)]

Mouse sperm (C57/BL6, 12 weeks old) were suspended in each of HTF Medium (manufactured by FUJIFILM Wako Pure Chemical Corporation) supplemented with RIM culture supernatant D3 at each concentration and HTF Medium supplemented with no culture supernatant as a negative control (control) in a water bath at 37°C, and these were each used as a sample. Subsequently, the sample was cultured for 1 hour at 37°C. Next, 3 µL of the sample was analyzed for the straight-line velocity (VSL) of all sperm observed by using a sperm motility analyzer (Computer Assisted Sperm Analysis (CASA) system), and the extent of increase in the median value for the medium supplemented with RIM culture supernatant D3 at each concentration based on the median value for the negative control is shown in Fig. 4.

As shown in Fig. 4, it was confirmed that the supplementation with the cell culture supernatant increased the median value and increased the motility of the sperm. A quite excellent effect was obtained when the concentration of the culture supernatant was 10% and 50%.

### [Example 5: Preparation of culture supernatant (2)]

Umbilical cord mesenchymal stem cells (C-12971 Human Mesenchymal Stem Cells from Umbilical Cord Matrix (hMSC-UC), manufactured by PromoCell) were cultured in each of RIM medium (serum-free medium for mesenchymal stem cells, containing EGF, bFGF, albumin, transferrin, and insulin, Rohto) and RS medium (serum-free medium for mesenchymal stem cells, containing EGF, bFGF, albumin, transferrin, and insulin, Rohto) for 3 days, RS medium or RIM medium was newly added to the cells after culture for 3 days, followed by culturing for 1 day, to obtain culture supernatants (hereinafter referred to as "RIM culture supernatant D1" and "RS culture supernatant D1," respectively). Various factors included in the culture supernatants were measured by ELISA, and it was confirmed that PGE2, IL-6, HGF, MCP1, MMP2, BDNF, and CD63+ exosomes were included.

### [Example 6: Motility of sperm (3)]

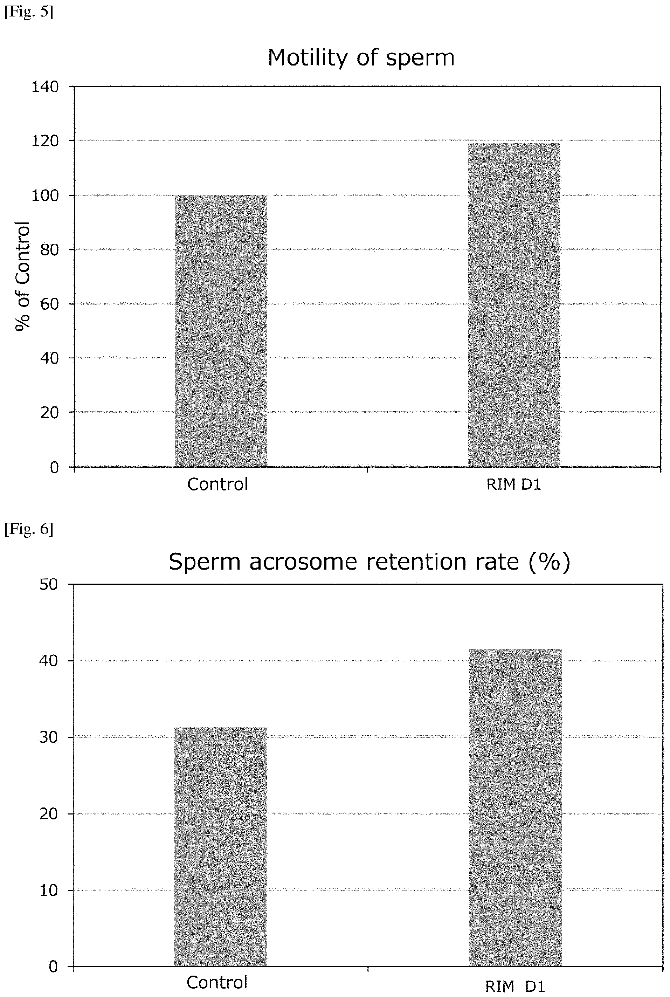

In the same manner as in Example 2, mouse sperm (C57/BL6, 12 weeks old) were suspended in HTF Medium (manufactured by FUJIFILM Wako Pure Chemical Corporation) supplemented with RIM culture supernatant D1 in such a way as to have a final concentration of 10% (also referred to as 10% RIM culture supernatant D1) and HTF Medium supplemented with no culture supernatant as a negative control (control), and these were each used as a sample to analyze the straight-line velocity (VSL) of all sperm. With the median value for the control set to 100, the median value for 10% RIM culture supernatant D1 is shown in Fig. 5.

As shown in Fig. 5, it was confirmed that the supplementation with the cell culture supernatant increased the median value and increased the motility of the sperm.

### [Example 7: Suppression of acrosome reaction of sperm (2)]

In the same manner as in Example 3, HTF Medium (manufactured by FUJIFILM Wako Pure Chemical Corporation) supplemented with RIM culture supernatant D1 in such a way as to have a final concentration of 10% (also referred to as 10% RIM culture supernatant D 1) and HTF Medium supplemented with no culture supernatant as a negative control (control) were used to analyze the proportion of sperm retaining an acrosome after culture for 60 minutes. The proportions of the number of positive sperm retaining an acrosome are shown in Fig. 6.

As shown in Fig. 6, it was confirmed that the supplementation with the cell culture supernatant remarkably suppressed the acrosome reaction. Therefore, it was revealed that the loss of fertility of sperm can be suppressed by supplementing a sperm medium such as HTF Medium with a cell culture supernatant.

### [Example 8: Fertilized egg acquisition rate]

Mouse sperm (C57/BL6, 12 weeks old, 1.0 × 10⁶ cells/mL) were suspended in HTF Medium (manufactured by FUJIFILM Wako Pure Chemical Corporation) supplemented with RIM culture supernatant D1 or D3 in such a way as to have a final concentration of 10% and HTF Medium supplemented with no culture supernatant as a negative control (control), and these were each used as a sample and then allowed to stand in an incubator (37°C) for 30 minutes or more after preparing solution. In addition, mouse sperm were suspended in a seminal vesicle gland fluid (fluid obtained by homogenizing the seminal vesicle gland in HTF Medium and containing 10 mg/mL of protein) as a positive target, and this was used as a sample and then allowed to stand in an incubator (37°C) for 30 minutes or more after preparing solution. 40 µL of these cultured sperm suspensions were injected into the uterus of 7-week-old female mice, respectively, induced to estrus by exogenous hormone administration, by using a gel loading tip. Twenty-four hours after injection of the sperm suspensions, fertilized embryos were collected from the fallopian tubes of the female mice, and the proportion of 2-cell stage embryos was measured. RIM culture supernatant D3 is a culture supernatant obtained in the same manner as in Example 1. The same applies to the following.

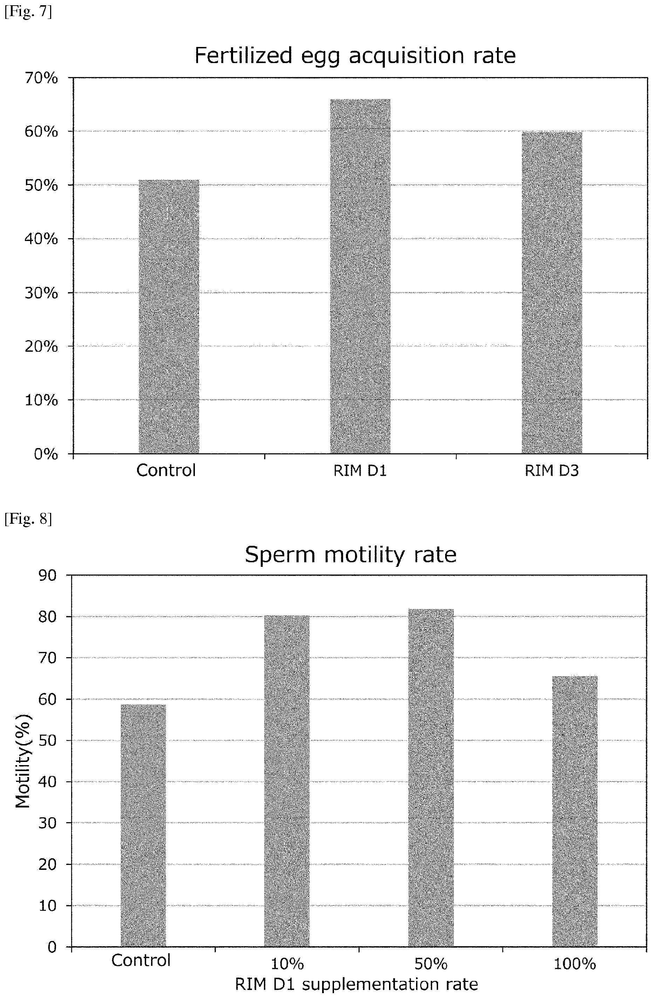

As shown in Fig. 7, it was confirmed that the supplementation with the cell culture supernatant increased the cleavage rate. Therefore, it was revealed that supplementation with a mesenchymal stem cell culture supernatant increases the motility of sperm and increases the acquisition rate of a fertilized egg.

### [Example 9: Motility of cattle sperm (1)]

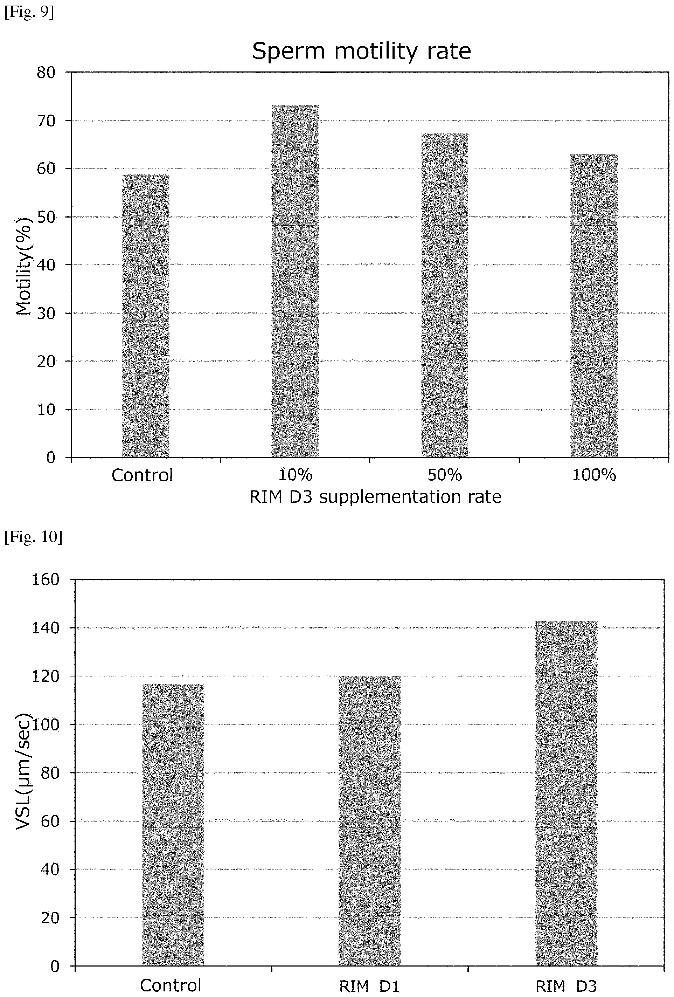

Frozen cattle semen purchased from the Livestock Improvement Association of Japan, Inc. was thawed by warming in a water bath at 37°C for 15 seconds, and then washed twice with HTF Medium (manufactured by FUJIFILM Wako Pure Chemical Corporation). The washed sperm were suspended in HTF Medium supplemented with RIM culture supernatant D1 or RIM culture supernatant D3 at each concentration and HTF Medium supplemented with no culture supernatant as a negative control, and these were each used as a sample. Subsequently, the sample was cultured for 1 hour at 37°C. Next, 3 µL of the sample was analyzed by using a sperm motility analyzer (Computer Assisted Sperm Analysis (CASA) system). The proportion of motile sperm in all sperm observed (Motility) was analyzed (Fig. 8 (RIMD1), Fig. 9 (RIMD3)).

As shown in Fig. 8 and Fig. 9, it was confirmed that also in cattle sperm, the supplementation with the cell culture supernatant increased the motility of the sperm. An especially good effect was obtained when the concentration of the culture supernatant was 10% and 50%.

### [Example 10: Motility of cattle sperm (2)]

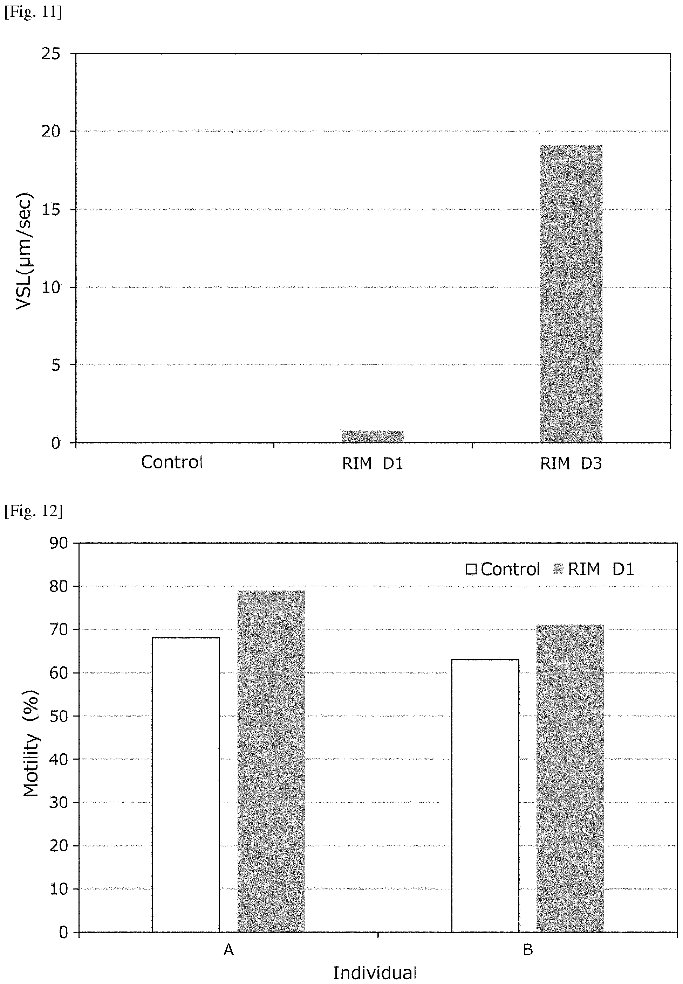

Frozen cattle semen was thawed by warming in a water bath at 37°C for 15 seconds, and then suspended in HTF Medium supplemented with RIM culture supernatant D1 or RIM culture supernatant D3 in such a way as to have a final concentration of 10% and HTF Medium (manufactured by FUJIFILM Wako Pure Chemical Corporation) supplemented with no supernatant as a negative control. Immediately after that, each of the sperm suspensions was washed once with HTF Medium, then the washed sperm were suspended again in HTF Medium, and this was used as a sample. Subsequently, the sample was cultured for 1 hour at 37°C. Next, 3 µL of the sample was analyzed for straight-line velocity (VSL) by using a sperm motility analyzer (Computer Assisted Sperm Analysis (CASA) system) (Fig. 10). In addition, the extent of increase in the median value for the medium supplemented with the culture supernatant based on the median value for the control is shown in Fig. 11.

As shown in Fig. 11, it was confirmed that the supplementation with the cell culture supernatant increased the median value and increased the motility of the sperm.

### [Example 11: Motility of pig sperm]

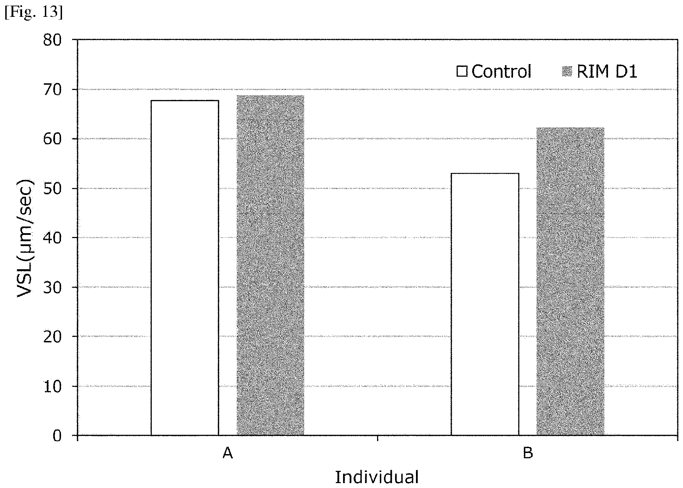

Fresh pig sperm (individual A, individual B) on day 5 after ejaculation diluted with HIRO-SWINE B Solution were washed twice with HTF Medium (manufactured by FUJIFILM Wako Pure Chemical Corporation). The washed sperm were suspended in HTF Medium supplemented with RIM culture supernatant D1 in such a way as to have a final concentration of 10% and HTF Medium supplemented with no culture supernatant as a negative control, and these were each used as a sample. Subsequently, the sample was cultured for 1 hour at 37°C. Next, 3 µL of the sample was analyzed by using a sperm motility analyzer (Computer Assisted Sperm Analysis (CASA) system). The proportion of motile sperm in all sperm observed (Motility, Fig. 12) and the straight-line velocity (VSL, Fig. 13) thereof were analyzed.

As shown in Fig. 12 and Fig. 13, it was confirmed that the supplementation with the cell culture supernatant increased the motility of the sperm.

### INDUSTRIAL APPLICABILITY

According to the present invention, sperm having high motility and suppressed sperm acrosome reaction can be provided. Sperm treated by the present invention have higher motility than and suppressed sperm acrosome reaction as compared with sperm treated by a conventional method, and thus have a remarkably higher fertilization success rate.

## Claims

1. A composition for treating sperm, comprising a mesenchymal stem cell culture supernatant.

2. The composition for treating sperm according to claim 1, wherein the composition is a sperm conditioning solution, a sperm dilution solution, a sperm preservation solution, an artificial insemination solution, an in vitro fertilization solution, a solution for improving sperm motility, or a solution for retaining sperm fertility.

3. The composition for treating sperm according to claim 1 or 2, wherein the mesenchymal stem cells are derived from adipose tissue, umbilical cord tissue, or bone marrow tissue.

4. The composition for treating sperm according to claim 1 or 2, wherein the composition is a mesenchymal stem cell culture supernatant obtained by culturing the mesenchymal stem cells in a serum-free medium.

5. An agent for improving sperm motility, comprising a mesenchymal stem cell culture supernatant.

6. An agent for retaining sperm fertility, comprising a mesenchymal stem cell culture supernatant.

7. A method for improving sperm motility, comprising culturing sperm in a solution comprising a mesenchymal stem cell culture supernatant.

8. A method for retaining sperm fertility, comprising culturing sperm in a solution comprising a mesenchymal stem cell culture supernatant.
